# EUROPEAN PATENT SPECIFICATION

(11) **EP 3 860 973 B1**
(45) Date of publication and mention of the grant of the patent: **09.04.2025**
(21) Application number: 19790705.8
(22) Date of filing: 03.10.2019
(51) Int. Cl.: C07C 227/40, C07C 229/16, C07C 229/76

(54) **AMORPHOUS FORM OF CHELATING AGENTS AND PROCESS FOR PREPARING THEM**
AMORPHE FORM VON CHELATBILDNERN UND VERFAHREN ZUR HERSTELLUNG DAVON
FORME AMORPHE D'AGENTS DE CHÉLATION ET PROCÉDÉ DE PRÉPARATION CORRESPONDANT

(30) Priority: 04.10.2018 PT 2018115056
(43) Date of publication of application: 11.08.2021
(73) Proprietor: Hovione Scientia Limited, Ringaskiddy, Co. Cork (IE)
(72) Inventor: SALDANHA, Susana, 1500-618 Lisboa (PT); PARREIRA, Antonio, 3870-030 Murtosa (PT); SEQUEIRA, Sara, 2710-235 Sintra (PT); ANTUNES, Rafael, 2900-005 Setubal (PT)
(74) Representative: Privett, Marianne Alice Louise
(86) International application number: PCT/GB2019/052799
(87) International publication number: WO 2020/070508

(56) References cited:
- US-A- 4 636 336
- JACOB P. THYSSEN: "Metal allergy from dermatitis to implant and device failure", 13 April 2018
- ABRAHAM CLEARFIELD ET AL: "METAL PHOSPHONATE CHEMISTRY: FROM SYNTHESIS TO APPLICATIONS", 1 January 2012
- CORROSION, 1 January 1991 (1991-01-01)
- SAITOH H ET AL: "METAL OXIDE POWDER SYNTHESIZED WITH AMORPHOUS METAL CHELATES", JOURNAL OF MATERIALS SCIENCE, KLUWER ACADEMIC PUBLISHERS, DORDRECHT, vol. 37, no. 20, 15 October 2002 (2002-10-15), pages 4315 - 4319, XP001130438, ISSN: 0022-2461, DOI: 10.1023/A:1020696301394
- E MIKHAILOV ET AL: "Atmospheric Chemistry and Physics Amorphous and crystalline aerosol particles interacting with water vapor: conceptual framework and experimental evidence for restructuring, phase transitions and kinetic limitations", ATMOS. CHEM. PHYS, 1 January 2009 (2009-01-01), pages 9491 - 9522, XP055651154, Retrieved from the Internet <URL:E Mikhailov ET AL>

## Description

### BACKGROUND OF THE INVENTION

### FIELD OF THE INVENTION

The present invention relates generally to new stable amorphous forms of chelating agents, to compositions and uses thereof, and to a process of producing stable amorphous chelating agents, especially particles thereof. More particularly, it relates to a process for producing amorphous chelating agents by dissolving them in a suitable solvent or mixture of solvents, optionally purifying the solution and isolating particles of essentially amorphous chelating agent by solvent removal, preferably by spray drying. Moreover, the amorphous particulates produced in accordance with the process of present invention present advantageous characteristics regarding particle size batch-to-batch consistency and solubility. These agents can be applied, for example, in the pharmaceutical field particularly in novel formulations having these excipients in the composition.

### DESCRIPTION OF THE PRIOR ART

Chelating agents are chemical compounds whose structures permit the attachment of their two or more donor atoms (or sites) to a single metal ion simultaneously (Flora et al. 2015). These molecules are also called chelants, chelators or sequestering agents. Some examples of chelating agents are ethylenediaminetetraacetic acid/edetic acid (EDTA), sodium edetate (Na-EDTA), disodium edetate (2Na-EDTA), dipotassium edetate (2K-EDTA), calcium disodium edetate (2NaCa-EDTA), trisodium edetate (3Na-EDTA), diethylenetriaminepentaacetic acid/pentetic acid (DTPA), diethylenetriaminepentaacetic acid calcium trisodium (3NaCa-DTPA), nitrilotriacetic acid (NTA), amino tris(methylenephosphonic acid) (ATMP), ethylenediamine tetramethylene phosphonic acid (EDTMP), 1-hydroxyethane 1,1-diphosphonic acid (HEDP), ethylenediaminedisuccinic acid (EDDS), iminodisuccinic acid (IDS) chitosan, hydroxamic acid, oxalic acid, galactaric acid, metaphosphoric acid, phytic acid, citric acid, fumaric acid, malic acid or maltol. Some of the most used chelating agents are EDTA, 2Na-EDTA, 2NaCa-EDTA and DTPA. The structural formulas are represented below.

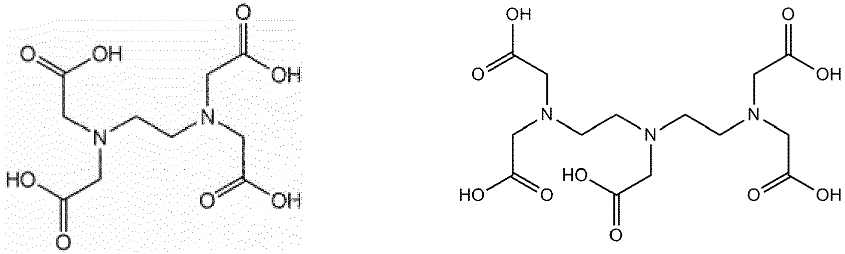

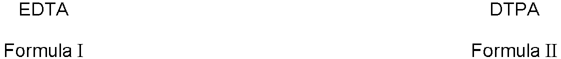

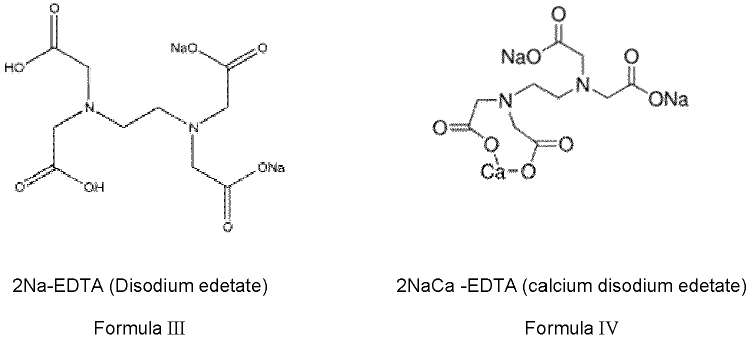

Chelating agents are not only used in industrial and agricultural applications, such as chemical water treatment, fertilizers, paper and textile production, cleaning and laundry operations, prevention/inhibition of the growth of microorganisms, soil remediation, wastes and effluents treatment, metal electroplating and other surface treatments, tanning processes, cement admixtures, photography, food products and cosmetics. They are also used in several medical applications such as chelation therapy to detoxify poisonous metal agents converting them into inert forms, as contrast agents in MRI (Magnetic Resonance Imaging) scanning, as radioisotope chelators and in pharmaceuticals as excipients or processing adjuvants.

In the pharmaceutical industry, chelating agents can be added to formulations to protect against autoxidation; they act by forming complexes with the heavy metal ions which are often required to initiate oxidative reactions (Loftsson. 2014) and to chelate metal leachables that arise from components and materials used in manufacturing, storage, and delivery of the therapeutic (Zhou et al, 2010). Chelating properties have major importance in biopharmaceuticals. Compared to small molecules, proteins in nature exhibit significantly lower stability due to the strong dependence of their physico-chemical properties on their structure and conformation. This and their generally larger-size offer multiple sites for potential interaction with leachables (like metal ions), increasing the risk for degradation and loss of activity. The metal chelator disodium EDTA (2Na-EDTA) has been commonly used in parenteral formulations. DTPA has also been used in several approved parenteral products (Zhou et al, 2010; FDA Inactive ingredient database). Chelating agents such as EDTA and DTPA are soluble in water only at higher pH (around 8) at room temperature which are conditions that could affect the structure and conformation of proteins.

There are several techniques for producing amorphous forms or amorphous solid dispersions namely, but not limited to, freeze-drying, precipitation, melt extrusion and spray-drying.

Spray drying is a well-established manufacturing technique which can be an effective strategy to deliver poorly water soluble drugs (Singh and Mooter, 2015). A typical spray-drying apparatus comprises a drying chamber, atomizing means for atomizing a solvent-containing feed into the drying chamber, a source of heated drying gas that flows into the drying chamber to remove solvent from the atomized-solvent-containing feed and product.

Spray drying of chelating agents is disclosed in:
U5958866 where a suspension of chelating agent and an alkaline earth metal sulphate is spray-dried. Nevertheless, in this case it is the complex of chelating agent with a metal that is spray-dried.

US4636336 which describes a process for reducing the volume of a liquid waste containing an organic amine chelating agent in which a finely atomized spray of liquid waste is contacted with a gas stream having a temperature in excess of thermal decomposition temperature of chelating agent. The objective of this process is to reduce the volume of radioactive waste produced and the drying temperature has to be higher than the thermal decomposition temperature of the chelating agent, meaning higher than 200°C (more precisely 250-400°C), much higher than the temperatures defined in the present invention (which are 50 to 100°C).

WO9929656 A1 where is described a method for producing high purity crystals of tetrasodium salt of ethylenediaminetetraacetic acid (4Na-EDTA). In this invention, a crystalline form is produced and those crystals could be recovered by spray drying, consuming organic solvents.

Journal of Materials Science, vol. 37, no 20 (2002-10-15), pages 4315-4319 which describes chelate powder consisting of amorphous particles that was synthesized through the process in which the droplet of the chelate solution is dried in the gas phase and solidified in a moment using a spray-dry technique. Two routes were studied: a conventional route of mechanically mixing crystalline metal-ethylenediaminetetraacetic acid (EDTA) powders and spray-dry mixing of metal-EDTA. These routes were followed by calcinations of metal-EDTA powder to form metal oxide powder.

Solid dispersions of the penta-ethyl ester prodrug of diethylenetriaminepentaacetic acid (DTPA) article describes the incorporation of a prodrug of DTPA into a matrix (Amorphous solid dispersion), by spray-drying. Nevertheless, this is a prodrug of DTPA and a polymer matrix is used to stabilize it (Yang et al., 2014).

There is no prior art describing a stable form of amorphous chelating agents such as EDTA (and its salts) or DTPA (and its salts) nor a process to obtain amorphous forms without the use of additives or of a support matrix (solid dispersion).

Currently the solution for the low solubility in water of chelating agents is the formation of salts. For example tetrasodium EDTA (4Na-EDTA) is highly soluble in water and disodium EDTA (2Na-EDTA) is slowly soluble in water. The higher the deprotonation level (more substituted carboxyls), the higher the solubility. Nevertheless, higher deprotonation leads to higher pH in solution, for instance 2Na-EDTA pH in solution ranges from 4 to 6, and 4Na-EDTA ranges from 10 to 11. The pH is critical to protein stability and should ideally be controlled to an optimal value (pH range 6 to 7) (Challener, 2015). Thus, it is highly desirable to have a salt less protonated which dissolves more and faster, from a formulation point of view.

### BRIEF DESCRIPTION OF THE DRAWINGS

Figure 1 illustrates the spray-drying set up used;
Figure 2 illustrates a SEM image of spray dried particles of amorphous DTPA;
Figure 3 illustrates a XRPD diffractogram of DTPA obtained by spray drying;
Figure 4 represents SEM image of spray dried particles amorphous EDTA;
Figure 5 illustrates a XRPD diffractogram of EDTA obtained by spray drying;Figure 6 represents SEM image of spray dried particles amorphous diammonium EDTA;
Figure 7 illustrates a XRPD diffractogram of diammonium EDTA obtained by spray drying;
Figure 8 represents a SEM image of spray dried particles amorphous 2Na-EDTA;
Figure 9 illustrates a XRPD diffractogram of 2Na-EDTA obtained by spray drying;
Figure 10 represents a graphic of crystalline 2Na-EDTA dissolution rate;
Figure 11 represents a graphic of amorphous 2Na-EDTA dissolution rate.

### DETAILED DESCRIPTION OF THE INVENTION

The present invention provides novel amorphous solid forms of chelating agents, such as EDTA and DTPA, and a method of preparing those amorphous forms. Surprisingly, we have found that the amorphous chelating agents can be obtained by a simple and industrial method comprising, for example, spray drying of a solution of chelating agents. Due to the spray drying technique used these amorphous forms have also a controlled particle size distribution.

In one aspect of this invention, amorphous chelating agents are provided and are suitable to be used in human and veterinary pharmaceutical applications. In another aspect of this invention, amorphous chelating agents are provided and are suitable to be used in biopharmaceutical formulations as well as pharmaceutical formulations.

The present inventors have now found that a stable amorphous form of chelating agents in a particulate form with controlled particle size would provide an advantageous solution due to its modified solubility profile. However, chelating agents such as, but not limited to, ethylenediaminetetraacetic acid/edetic acid (EDTA), diethylenetriamine-pentaacetic acid/pentetic acid (DTPA), nitrilotriacetic acid (NTA), amino tris(methylenephosphonic acid) (ATMP), ethylenediamine tetramethylene phosphonic acid (EDTMP), 1-hydroxyethane 1,1-diphosphonic acid (HEDP), ethylenediaminedisuccinic acid (EDDS), iminodisuccinic acid (IDS) hydroxamic acid, oxalic acid, galactaric acid, metaphosphoric acid or phytic acid and salts thereof have never been reported to be isolated in a stable amorphous form.

According to one aspect of the present invention, there is provided an amorphous form of a chelating agent according to claim 1. The amorphous form is stable, and may be provided in a "pure" state, without the need for any other compounds to provide stability.

Preferably, the amorphous form of a chelating agent is provided in particulate form.

Preferably, the chelating agent comprises ethylenediaminetetraacetic acid/edetic acid (EDTA) or a salt thereof, or diethylenetriaminepentaacetic acid/pentetic acid (DTPA), or a salt thereof, although we have found that other chelating agents may also be provided in amorphous form.

Preferably, the amorphous form is obtained by spray drying, but other suitable techniques may be used. The invention thus provides a spray dried amorphous form of a chelating agent. A spray dried amorphous form of ethylenediaminetetraacetic acid/edetic acid (EDTA) or a salt thereof, or a spray dried amorphous form of diethylenetriaminepentaacetic acid/pentetic acid (DTPA), or a salt thereof, are particularly preferred.

Suitably, the chelating agent may comprise ethylenediaminetetraacetic acid/edetic acid (EDTA), diethylenetriaminepentaacetic acid/pentetic acid (DTPA), nitrilotriacetic acid (NTA), amino tris(methylenephosphonic acid) (ATMP), ethylenediamine tetramethylene phosphonic acid (EDTMP ethylenediaminedisuccinic acid (EDDS), iminodisuccinic acid (IDS), or a salt of any one or more of the above acids, or a mixture of two or more of any of the above acids or salts thereof. We have found these compounds may be provided in amorphous form. Preferably, they are spray dried.

We have found that, quite surprisingly, the present invention provides chelating agents without the need for any additional compounds or additives to stabilise the amorphous form, and that they are stable. The invention thus provides an isolated amorphous form of a chelating agent, free of additives. It also provides an isolated amorphous form of a chelating agent, free of metal or metal-containing compounds, such as metal ions or metal salts.

We have also found that, quite surprisingly, the present invention provides chelating agents without the need for any support matrix (such as, for example, a polymer matrix) to stabilise the amorphous form, and that they are stable. The invention thus provides an isolated amorphous form of a chelating agent, free of a support matrix, such as a polymer matrix.

Preferably the chelating agent comprises ethylenediaminetetraacetic acid/edetic acid (EDTA), sodium edetate (Na-EDTA), disodium edetate (2Na-EDTA), dipotassium edetate (2K-EDTA), calcium disodium edetate (2NaCa-EDTA), trisodium edetate (3Na-EDTA), diethylenetriaminepentaacetic acid/pentetic acid (DTPA), pentasodium pentetate, or calcium trisodium pentetate, or a mixture of two or more of any of the above acids or salts.

The invention also provides an amorphous form of a chelating agent as described above, wherein the solubility in water of the amorphous form is higher than that of the corresponding crystalline form. This is illustrated further below in the Examples, and is a particular advantage of the amorphous chelating agents provided by the invention. The amorphous chelating agents provided herein also, in general, possess a higher dissolution rate in aqueous media such as water, and this is a further advantage.

In a further aspect, the invention provides a process for preparing an amorphous form of a chelating agent, which process comprises the steps of:
a) Dissolving the chelating agent in a suitable solvent to form a solution
b) Optionally purifying the solution;
c) Isolating an amorphous form of the chelating agent, wherein the amorphous form is free of any additional compounds or a support matrix;
d) Optionally post drying or conditioning the amorphous form of the chelating agent.

In the process of the invention, any suitable chelating agent listed in claim 1 may be used.

The present invention thus provides a process for the preparation of amorphous chelating agents comprising the steps of:
a) Preparing a solution of the chelating agent in a suitable solvent;
b) Isolating the amorphous form of the chelating agent.

Preparing a solution of chelating agent in step a) comprises the addition of crystalline chelating agent to a suitable solvent. Crystalline forms of the chelating agents may be readily obtained, as will be clear to those skilled in this field.

The solvent temperature used to prepare a solution of the chelating agent is suitably from about 20°C to about 60°C. The chelating agent can be dissolved in any suitable solvent, and suitable solvents include any solvents that have no adverse effect on the compound and can dissolve the starting material to a useful extent. Example of such solvents include, but are not limited to: water, aqueous solution of sodium hydroxide, aqueous solution of ammonia, or a mixture of two or more such solvents. Alternatively, or in addition, the solvent may be an organic solvent and may comprise, for example methanol, ethanol, isopropanol, or acetone or a mixtures of two or more such solvents.

Any appropriate chelating agent concentration may be used, up to the solubility limit. However a solution concentration of from about 0.05 % to about 10 % w/w is preferred, ideally between 0.5 % and 5.0% w/w where "%w/w" refers to the mass of the chelating agent as a percentage of the mass of the total solution. The concentration to be employed will generally be limited by the solubility of the chelating agent in the solvent.

Step a) may optionally include a purification step of the compound using, for example, a resin, activated charcoal, filtration or other suitable method.

Step b) involves isolating an amorphous form of the chelating agent from the solution obtained in step a). Isolation of the amorphous form of the chelating agent comprises the removal of the solvent by any known technique, such as freeze-drying, vacuum drying or spray-drying, preferably by spray drying. After step b), optionally, a post drying or conditioning step may be carried out.

Spray drying may be performed using any suitable or commercially available equipment.

A variety of atomization methods can be used, depending on the equipment chosen. For example, we have found a pneumatic spray nozzle orifice of 1.4 mm is suitable although alternative atomization methods such as two- or three-fluid, rotary, pressure and ultrasonic nozzles may be employed.

The preferential atomization gas flow in terms of liters per hour can be adjusted to the equipment in use and any suitable atomization gas flow can be used. Typically, for a small scale unit, 150 to 300 milliliters per hour is preferred. In an industrial scale a different flow may be used. In a preferred embodiment, the nozzle assembly can be cooled with a suitable fluid during spray drying to minimize product degradation. Any suitable drying temperature can be used.The outlet temperature range may be from about 20° C to about 220°C, preferably from about 40°C to about 80° C and more preferably from about 50°C to about 70°C.

As will be understood, the inlet temperature may be adjusted to attain the desired outlet temperature.

Any suitable solution flow rate can be used. For a small scale unit, the solution flow rate may preferably be from about 1 to about 20 ml/min, more preferably 2 to 15 ml/min for the 1.4 mm nozzle. For an industrial scale, the solution flow rate may be adjusted depending on the selected nozzle.

The drying gas flow rate for a small scale spray dryer may suitably be from about 20 kg/h to about 120 kg/h, preferably from about 40 kg/h to about 80 kg/h, most preferably about 40 kg/h. The drying gas flow rate for a larger spray dryer may, for example, be greater than about 120 kg/h preferably from about 360 kg/h to about 1250 kg/h, for example about 650 kg/h or about 1250 kg/h.

The outlet temperature, atomization flow rate, solution concentration and solution flow rate, among other tested parameters, can be combined and adjusted to obtain a compound with suitable quality.

The compounds obtained using the method of this invention are amorphous solids that shows stability over time.

Preferably the process of the invention is carried out such that the amorphous chelating agent particle size distribution is controlled. By using a spray drying technique for solvent evaporation we can adjust the droplet size and solution concentration in order to obtain the desired particle size. The range usually defined for this type of drying technique is below about 25 µm and above 1 µm of average particle size distribution. For example, average particle size distribution ranges of 1-20 µm, or 2-18 µm, or 3-15 µm may be used.

In a further aspect, the invention provides the use of an amorphous chelating agent according to the invention described herein, in a process or composition in the industrial, agricultural or domestic field.

For example, such use in a process or composition in the industrial, agricultural or domestic field may comprise: pulp bleaching, food and beverage preservation, industrial cleaning, detergents, cement, personal care and cosmetics, fertilizers, water treatment, or removal of and deactivation of metal ions in textiles.

The amorphous chelating agents of the present invention may be used in a conventional way in the pharmaceutical and biopharmaceutical field, in a similar manner to the way conventional chelating agents are used. Thus, in a further aspect, the invention also provides a pharmaceutical or biopharmaceutical composition comprising:
a) an active pharmaceutical ingredient (API), and
b) an amorphous chelating agent as disclosed herein.

Certain specific aspects and embodiments of the present invention will be explained in more detail with reference to the following examples. Example 1, 2, 3 and 4 are set forth to illustrate and aid in understanding the invention but are not intended to, and should not be considered to, limit its scope in any way. The experiments reported were carried out using a BUCHI model B-290 advanced spray dryer.

### Example 1: Amorphous DTPA production by spray drying

*Chelating agent (DTPA) solution preparation:*
DTPA in a mass proportion of 0.5% (w/w), was dissolved in water at 40°C, and stirred until a clear solution was obtained.

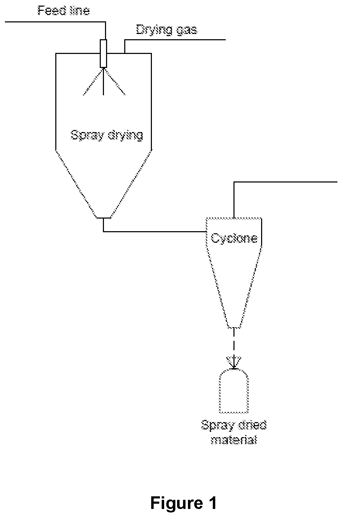

*Isolation of chelating agent (DTPA) amorphous particles:*
A lab scale spray dryer (Büchi, model B-290), equipped with a two fluid nozzle, was used to atomize and dry the solution. Co-current nitrogen was used to promote the drying after atomization. The spray drying unit was operated in open cycle mode (i.e., without recirculation of the drying gas). Figure 1 schematically shows the spray drying set up used.

Before feeding the solution to the nozzle, the spray drying unit was stabilized with nitrogen to assure stable inlet (T_in) and outlet temperatures (T_out). After stabilization, the solution was fed to the nozzle by means of a peristaltic pump, and atomized at the tip of the nozzle. The droplets were then dried in the spray drying chamber by current nitrogen. The stream containing the dried particles was directed into a cyclone and collected at the bottom. The main operating parameters during the spray drying process are summarized in table 1.

**Table 1 - Summary of the main operating conditions**

| **DTPA solution** | | |
|---|---|---|
| DTPA | 2.8 | g |
| Water | 550 | ml |

| **Spray Drying parameters** | | |
|---|---|---|
| T_in | 97 | °C |
| T_out | 60 | °C |
| F_drying (N2) | 40 | kg/h |
| Rotameter level | 40 | mm |

The compound obtained using the method of this invention is an amorphous solid with higher solubility and dissolution rate (in aqueous media) compared with the corresponding crystalline form. Several tests confirm its amorphous form, such as x-ray powder diffraction (XRPD) or differential scanning calorimetry (DSC).

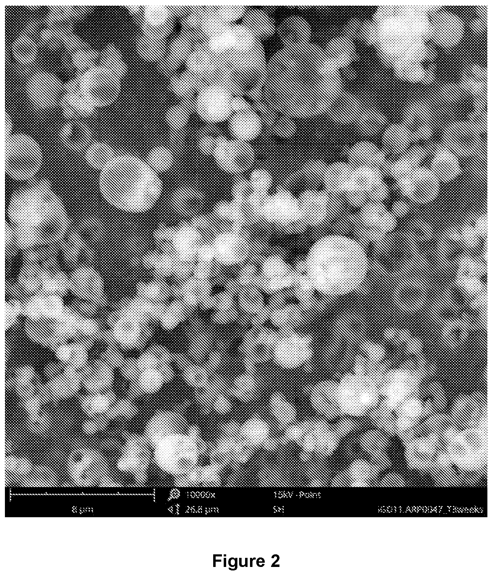

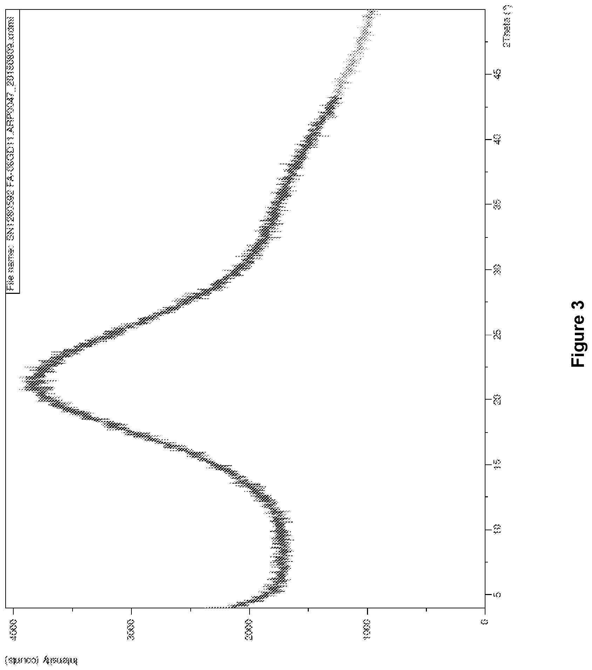

The appearance of the atomized material was characterized by means of scanning electron microscopy (SEM). A representative image of the particles obtained is shown in figure 2. The X-ray powder diffraction pattern of DTPA obtained by spray drying according to the process herein disclosed is presented in figure 3. The XRPD method settings applied for analyzing the sample are given in the table below:

| **Settings** | | |
|---|---|---|
| Sample stage mode | | Spinning |
| Spinner revolution time | | 1.000 |

| **Incident beam path** | | |
|---|---|---|
| PreFIX Module | | Progr. divergence slit with anti-scatter slit |
| | Soller slit | Soller slits 0.04 rad. |
| | Mask | Fixed incident beam mask 15 mm |
| | Divergence slit | Programmable divergence slit |
| | Anti-scatter slit | Fixed slit 1/4° |
| | Filter | None |
| | Beam attenuator | Actual |
| Beam knife | | None |
| Diffractead beam path | | Diffracted beam path1 |
| PreFIX Module | | PIXcel1-Medipix3 with PASS |
| Filter | | Large beta-filter Nickel |
| Soller slit | | Large Soller slits 0.04 rad. |
| Anti-scatter slit | | Programmable anti-scatter slit |
| Detector | | PIXcel1 D-Medipix3 detector |
| Beam attenuator | | Actual |
| Receiving slit | | None |

| **Measurement parameters** | | |
|---|---|---|
| Scan axis | | Gonio |
| Scan mode | | Continuous |
| Start angle (°): | | 3.9915 |
| End angle (°): | | 50.000 |
| Time per step (s): | | 39.525 |

Solubility method and classification are stated in the European Pharmacopeia (Ph. Eur.), chapter *5.11. Characters section in monographs.* This was used in the tests below. The results of a solubility test according to European Pharmacopoeia (Ph. Eur.) and presented in table 2 show a higher solubility of amorphous DTPA when compared to crystalline DTPA.

**Table 2 - DTPA solubility according to Ph. Eur., at 22°C**

| **Volume (mi)** | **Crystalline Industrial grade** | **Crystalline pharma grade** | **Amorphous pharma grade** | **Ph. Eur. Classification** |
|---|---|---|---|---|
| 0.1 | No | No | No | Very soluble |
| 1.0 | No | No | No | Freely soluble |
| 3.0 | No | No | No | Soluble |
| 10.0 | No | No | No | Sparingly soluble |
| 20.0 | No | No | No | Slightly soluble |
| 30.0 | No | No | **YES** | Very slightly soluble |
| 40.0 | No | No | -- | Insoluble |
| 50.0 | No | No | -- | Insoluble |
| 60.0 | No | No | -- | Insoluble |
| 70.0 | No | No | -- | Insoluble |
| 80.0 | **YES** | **YES** | -- | Insoluble |
| Mass | 100 mg | | | -- |

Solubility was assessed using the European Pharmacopeia method referred to above where increasing amounts of water are added to 100 mg of powder until its complete dissolution (or otherwise). According to this method if 100 mg do not solubilize in 30 ml the material is considered insoluble. Nevertheless, in this case, increasing amounts of water were added after 30 ml, in order to identify the actual solubility of the material.

### Example 2: Amorphous EDTA production by spray drying

*Chelating agent (EDTA) solution preparation:*
EDTA in a mass proportion of 0.05 % (w/w), was dissolved in water at 60°C, and stirred until a clear solution was obtained.

*Isolation of chelating agent (EDTA) amorphous particles:*
A lab scale spray dryer (Büchi, model B-290), equipped with a two fluid nozzle, was used to atomize and dry the solution. Co-current nitrogen was used to promote the drying after atomization. The spray drying unit was operated in open cycle mode (i.e., without recirculation of the drying gas). Figure 1 schematically showsthe spray drying set up used.

Before feeding the solution to the nozzle, the spray drying unit was stabilized with nitrogen to assure stable inlet (T_in) and outlet temperatures (T_out). After stabilization, the solution was fed to the nozzle by means of a peristaltic pump, and atomized at the tip of the nozzle. The droplets were then dried in the spray drying chamber by co-current nitrogen. The stream containing the dried particles was directed into a cyclone and collected at the bottom. The main operating parameters during the spray drying process are summarized in table 3.

**Table 3 - Summary of the main operating conditions**

| **EDTA solution** | | |
|---|---|---|
| EDTA | 2.3 | g |
| Water | 5000 | ml |

| **Spray Drying parameters** | | |
|---|---|---|
| T_in | 188 | °C |
| T_out | 80 | °C |
| Rotameter level | 50 | mm |

The compound obtained using the method of this invention is an amorphous solid with higher solubility and dissolution rate compared with the corresponding crystalline form. Several tests confirm its amorphous form, such as x-ray powder diffraction (XRPD) or differential scanning calorimetry (DSC).

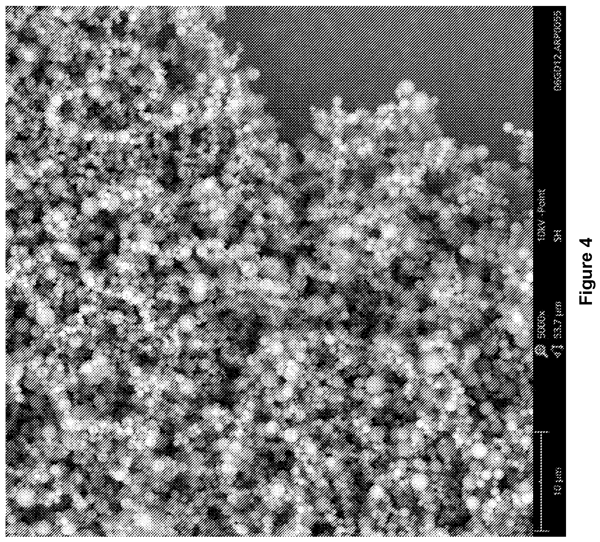

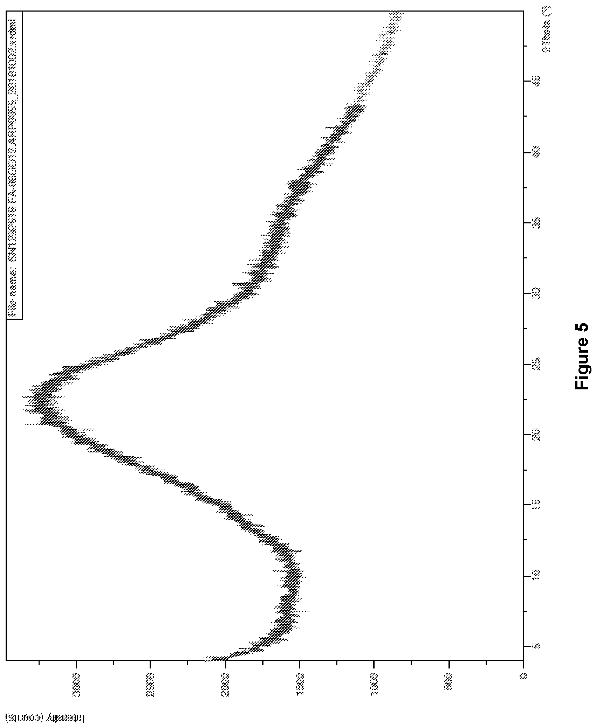

The appearance of the atomized material was characterized by means of scanning electron microscopy (SEM). A representative image of the particles obtained is shown in figure 4. The X-ray powder diffraction pattern of EDTA obtained by spray drying according to the process herein disclosed is presented in figure 5. The method used to analyze the samples is as described in Example 1.

Solubility method and classification are stated in the European Pharmacopeia (Ph. Eur.), chapter *5.11. Characters section in monographs.* The results of solubility test according to European Pharmacopoeia (Ph. Eur.) presented in table 4 show higher solubility of amorphous EDTA when compared to crystalline EDTA.

**Table 4 - EDTA solubility according to Ph. Eur., at 22°C**

| **Volume (ml)** | **Crystalline reagent grade** | **Amorphous pharma grade** | **Ph. Eur. Classification** |
|---|---|---|---|
| 0.1 | No | No | Very soluble |
| 1.0 | No | No | Freely soluble |
| 3.0 | No | No | Soluble |
| 10.0 | No | No | Sparingly soluble |
| 20.0 | No | No | Slightly soluble |
| 50.0 | No | No | Very slightly soluble |
| 100.0 | No | No | Insoluble |
| 200.0 | No | No | Insoluble |
| 250.0 | No | **YES** | Insoluble |
| 300.0 | No | -- | Insoluble |
| 5000 | No* | -- | Insoluble |

| | | | |
|---|---|---|---|
| *According to bibliography solubility in water: 1 to 500 | | | |

The solubility was tested (with 100mg of powder) as per the European Pharmacopeia test as described in Example 1.

### Example 3: Amorphous diammonium EDTA production by spray drying

*Chelating agent (2NH4-EDTA) solution preparation:*
EDTA in a mass proportion of 0.5 % (w/w), was dissolved in water with 0.4% ammonium hydroxide at 22°C, and stirred until a clear solution was obtained.

*Isolation of chelating agent (2NH4-EDTA) amorphous particles:*
A lab scale spray dryer (Büchi, model B-290), equipped with a two fluid nozzle, was used to atomize and dry the solution. Co-current nitrogen was used to promote the drying after atomization. The spray drying unit was operated in open cycle mode (i.e., without recirculation of the drying gas). Figure 1 schematically showsthe spray drying set up used.

Before feeding the solution to the nozzle, the spray drying unit was stabilized with nitrogen to assure stable inlet (T_in) and outlet temperatures (T_out). After stabilization, the solution was fed to the nozzle by means of a peristaltic pump, and atomized at the tip of the nozzle. The droplets were then dried in the spray drying chamber by co-current nitrogen. The stream containing the dried particles was directed into a cyclone and collected at the bottom. The main operating parameters during the spray drying process are summarized in table 5.

**Table 5 - Summary of the main operating conditions**

| **EDTA solution** | | |
|---|---|---|
| EDTA | 2.8 | g |
| Water | 550 | ml |
| Ammonium hydroxide (30%w/v) | 2.2 | ml |

| **Spray Drying parameters** | | |
|---|---|---|
| T_in | 97 | °C |
| T_out | 60 | °C |
| F_drying (N₂) | 40 | kg/h |
| Rotameter level | 40 | mm |

The compound obtained using the method of this invention is an amorphous solid with higher solubility and dissolution rate compared with the corresponding crystalline form. Several tests confirm its amorphous form, such as x-ray powder diffraction (XRPD) or differential scanning calorimetry (DSC).

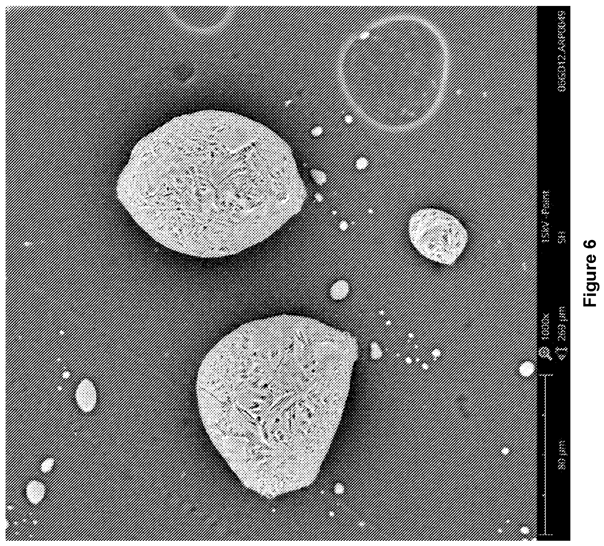

The appearance of the atomized material was characterized by means of scanning electron microscopy (SEM). A representative image of the particles obtained is shown in figure 6.

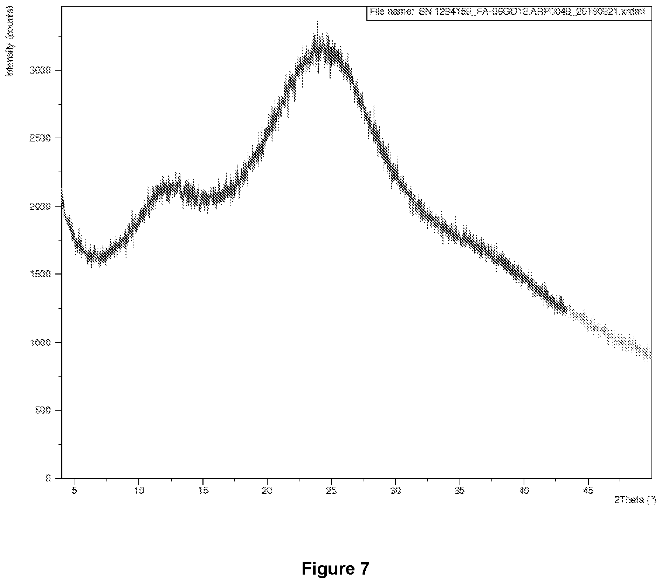

The X-ray powder diffraction pattern of diammonium EDTA obtained by spray drying according to the process herein disclosed is presented in figure 7. The method used to analyze the samples is as described in Example 1.

Solubility method and classification are stated in the European Pharmacopeia (Ph. Eur.), chapter *5.11. Characters section in monographs.*

The results of solubility test according to European Pharmacopoeia (Ph. Eur.) presented in table 6 show higher solubility of amorphous EDTA obtained when compared to crystalline EDTA.

**Table 6 - EDTA solubility according to Ph. Eur., at 22°C**

| **Volume (mi)** | **Crystalline reagent grade** | **Amorphous pharma grade** | **Ph. Eur. Classification** |
|---|---|---|---|
| 0.1 | No | No | Very soluble |
| 1.0 | No | No | Freely soluble |
| 3.0 | No | No | Soluble |
| 10.0 | No | **YES** | Sparingly soluble |
| 20.0 | No | -- | Slightly soluble |
| 30.0 | No | -- | Very slightly soluble |
| 40.0 | No | -- | Insoluble |
| 50.0 | No | -- | Insoluble |
| 60.0 | No | -- | Insoluble |
| 70.0 | No | -- | Insoluble |
| 80.0 | No | -- | Insoluble |
| 90.0 | No | -- | Insoluble |
| 100.0 | No* | -- | Insoluble |

| | | | |
|---|---|---|---|
| *According to bibliography solubility in water: 1 to 500 | | | |

The solubility was tested (with 100mg of powder) as per the European Pharmacopeia test as described in Example 1.

### Example 4: Amorphous 2Na-EDTA production by spray drying

*Chelating agent (2Na-EDTA) solution preparation:*
2Na-EDTA in a mass proportion of 0.7% (w/w), was dissolved in water at 22°C, and stirred until a clear solution was obtained.

*Isolation of chelating agent (2Na-EDTA) amorphous particles:*
A lab scale spray dryer (Büchi, model B-290), equipped with a two fluid nozzle, was used to atomize and dry the solution. Co-current nitrogen was used to promote the drying after atomization. The spray drying unit was operated in open cycle mode (i.e., without recirculation of the drying gas). Figure 1 schematically describes the spray drying set up used.

Before feeding the solution to the nozzle, the spray drying unit was stabilized with nitrogen to assure stable inlet (T_in) and outlet temperatures (T_out). After stabilization, the solution was fed to the nozzle by means of a peristaltic pump, and atomized at the tip of the nozzle. The droplets were then dried in the spray drying chamber by co-current nitrogen. The stream containing the dried particles was directed into a cyclone and collected at the bottom. Main operating parameters during the spray drying process are summarized in table 7.

**Table 7 - Summary of the main operating conditions**

| **2Na-EDTA solution** | | |
|---|---|---|
| EDTA-2Na | 2.8 | g |
| Water | 400 | ml |

| **Spray Drying parameters** | | |
|---|---|---|
| T_in | 101 | °C |
| T_out | 70 | °C |
| F_drying (N2) | 40 | kg/h |
| Rotameter level | 48 | mm |

The compound obtained using the method of this invention is an amorphous solid with higher solubility and dissolution rate compared with the corresponding crystalline form. Several tests confirm its amorphous form, such as x-ray powder diffraction (XRPD) or differential scanning calorimetry (DSC).

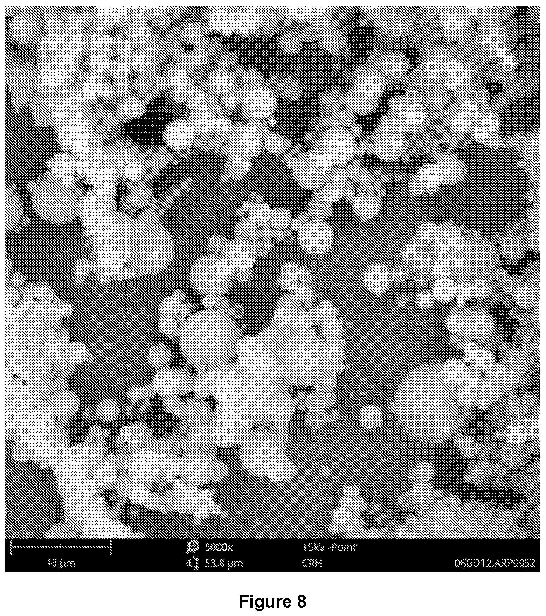

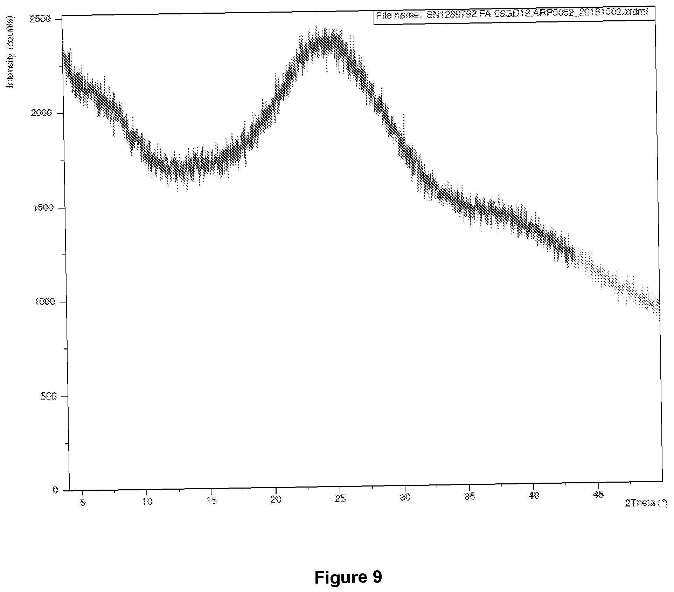

The appearance of the atomized material was characterized by means of scanning electron microscopy (SEM). A representative image of the particles obtained is shown in figure 8. The X-ray powder diffraction pattern of 2Na-EDTA obtained by spray drying according to the process herein disclosed is presented in figure 9. The method used to analyze the samples is as described in Example 1.

Solubility method and classification are stated in the European Pharmacopeia (Ph. Eur.), chapter *5.11. Characters section in monographs.*

The results of solubility test according to European Pharmacopoeia (Ph. Eur.) presented in table 8 show the solubility of amorphous 2Na-EDTA and crystalline 2Na-EDTA.

**Table 8 - 2Na-EDTA solubility according to Ph. Eur., at 22°C**

| **Volume (ml)** | **Crystalline reagent grade** | **Amorphous pharma grade** | **Ph. Eur. Classification** |
|---|---|---|---|
| 0.1 | No | No | Very soluble |
| 1.0 | No | No | Freely soluble |
| 3.0 | **YES** | **YES** | Soluble |

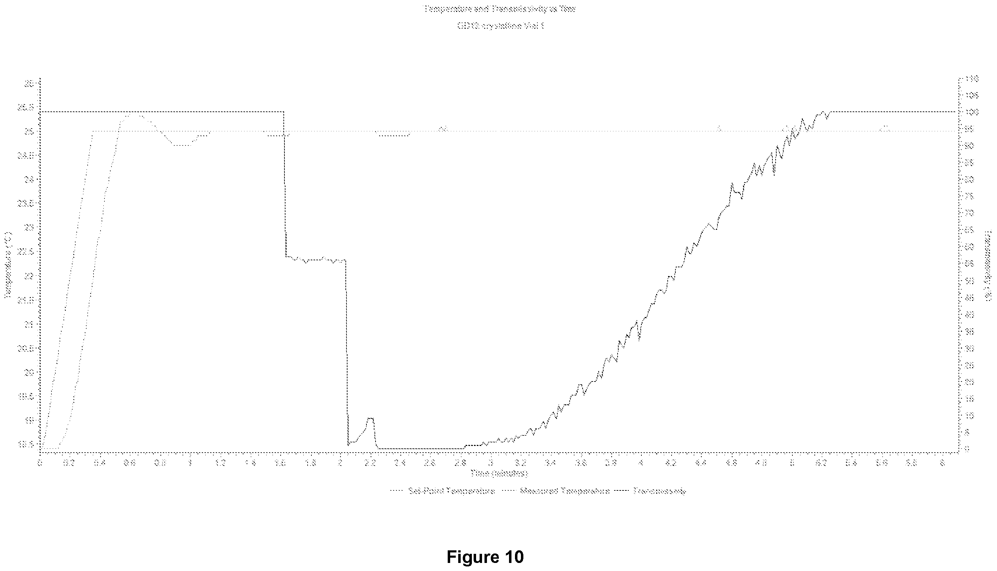

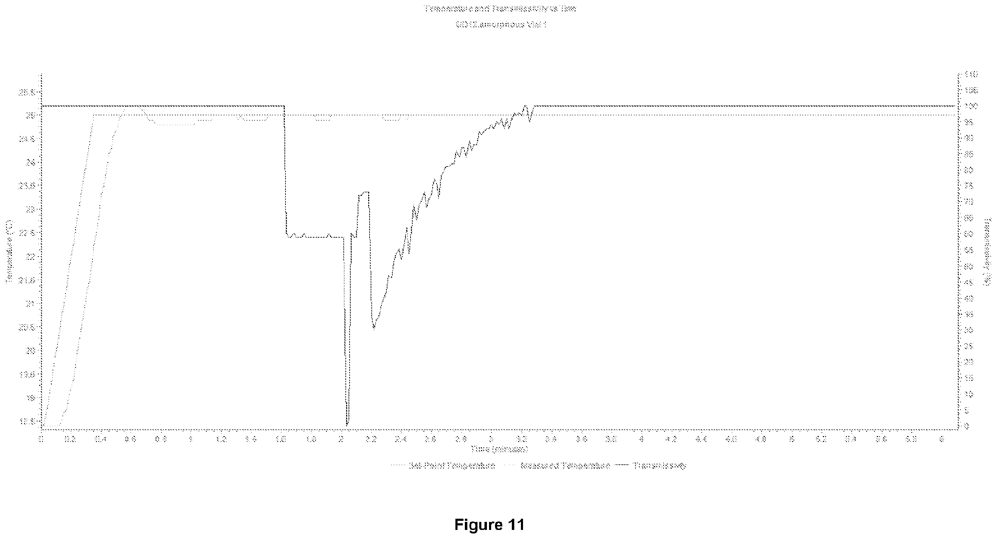

Since both morphologic forms are soluble, the dissolution rate was tested. The dissolution time for dissolving 250 mg in 5 ml of each form was assessed and the results are presented in table 9. The test was performed using the equipment Crystalline^{®}. This equipment reads the transmittance of a liquid: transmittances of 100% mean that there is a solution, and that the powder is completely dissolved. The powder was added to the vessel filled with water (0.5% w/w concentration) in the equipment and the time to reach a transmittance of 100% was measured. The results of the crystalline and amorphous forms are presented in figures 10 and 11, respectively.

**Table 9 - 2Na-EDTA dissolution rate, at 25°C**

| | **Dissolution rate** |
|---|---|
| **2Na-EDTA Crystalline reagent grade** | 3 min |
| **2Na-EDTA Amorphous pharma grade** | 1 min |

### REFERENCES:

FLORA, Govinder, MITTAL, Megha, FLORA, Swaran, Medical Countermeasures-Chelation Therapy, Handbook of Arsenic Toxicology, 2015.
LOFTSSON, Thorstein, Degradation Pathways, Drug stability for pharmaceutical scientists, 2014.
ZHOU, Shuxia, Lewis Lavinia, Singh Satish, Metal Leachables in therapeutic biologic products: Origin, Impact and Detection, 2010.
SINGH A, MOOTER Van den, Spray drying formulation of amorphous solid dispersions, 2016.
YANG, Yu-Tsai, DI Pasqua Anthony, Zhang Yong, Sued Katsihiko, Jay Michael, Solid dispersions of the penta-ethyl ester prodrug of diethylenetriaminepentaacetic acid (DTPA): formulation design and optimization studies, 2014.
CHALLENER, Cynthia A., Excipient selection for protein stabilization: The complex task of stabilizing proteins is made more challenging due to the limited number of approved excipients, 2015.
JOURNAL OF MATERIALS SCIENCE, vol 37, no. 20, 15 October 2002 (2002-10-15) pages 4315-4319, XP001130438

## Claims

1. An amorphous form of a chelating agent selected from:
a) ethylenediaminetetraacetic acid/edetic acid (EDTA), sodium edetate (Na-EDTA), disodium edetate (2Na-EDTA), dipotassium edetate (2K-EDTA), calcium disodium edetate (2NaCa-EDTA), trisodium edetate (3Na-EDTA), diammonium edetate ((NH₄)₂-EDTA), or a salt of any one or more of the above; or
b) diethylenetriaminepentaacetic acid/pentetic acid (DTPA), pentasodium pentetate, calcium trisodium pentetate, nitrilotriacetic acid (NTA), amino tris(methylenephosphonic acid) (ATMP), ethylenediamine tetramethylene phosphonic acid (EDTMP), ethylenediaminedisuccinic acid (EDDS), iminodisuccinic acid (IDS), or a salt of any one or more of the above acids, or
c) a mixture of two or more of any of the above acids or salts thereof selected from (a) or (b);
wherein the amorphous form is free of any additional compounds or a support matrix.

2. An amorphous form according to claim 1, wherein the chelating agent comprises ethylenediaminetetraacetic acid/edetic acid (EDTA), sodium edetate (Na-EDTA), disodium edetate (2Na-EDTA), dipotassium edetate (2K-EDTA), calcium disodium edetate (2NaCa-EDTA), trisodium edetate (3Na-EDTA), diammonium edetate ((NH₄)₂-EDTA), diethylenetriaminepentaacetic acid/pentetic acid (DTPA), pentasodium pentetate, or calcium trisodium pentetate, or a mixture of two or more of any of the above acids or salts.

3. A process for preparing an amorphous form of a chelating agent according to any preceding claim, which process comprises the steps of:
a) Dissolving the chelating agent in a suitable solvent to form a solution;
b) Optionally purifying the solution;
c) Isolating an amorphous form of the chelating agent, wherein the amorphous form is free of any additional compounds or a support matrix;
d) Optionally post drying or conditioning the amorphous form of the chelating agent.

4. A process according to claim 3, wherein the solution is purified using one or more resins, activated charcoal, or by filtration.

5. A process according to any one of claims 3 or 4, wherein the solvent is: water, an aqueous solution of sodium hydroxide, an aqueous solution of ammonia solution, or a mixture of two or more of these solvents.

6. A process according to claim 5, wherein an organic solvent may be added, optionally wherein the additional solvent is an alcohol or acetone.

7. A process according to any one of claims 3 to 6, wherein isolating an amorphous form of the chelating agent is carried out by solvent removal.

8. A process according to any one of claims 3 to 7, wherein a solvent removal technique such as freeze-drying, vacuum drying or spray-drying is used.

9. A process according to according to any one of claims 3 to 8, wherein a post drying or conditioning step is be performed.

10. A process according to according to any one of claims 3 to 9, wherein the amorphous chelating agent particle size distribution is controlled.

11. Use of an amorphous chelating agent according to any one of claims 1 or 2 in a process or composition in the industrial, agricultural or domestic field.

12. Use according to claim 11, wherein the field comprises: pulp bleaching, food and beverage preservation, industrial cleaning, detergents, cement, personal care and cosmetics, fertilizers, water treatment, or removal of and deactivation of metal ions in textiles.

13. A pharmaceutical or biopharmaceutical composition comprising:
a) an active pharmaceutical ingredient, and
b) an amorphous chelating agent according to any one of claims 1 or 2.

## Patentansprüche

1. Amorphe Form eines Chelatbildners, ausgewählt aus:
a) Ethylendiamintetraessigsäure/Edetinsäure (EDTA), Natriumedetat (Na-EDTA), Dinatriumedetat (2Na-EDTA), Dikaliumedetat (2K-EDTA), Calciumdinatriumedetat (2NaCa-EDTA), Trinatriumedetat (3Na-EDTA), Diammoniumedetat ((NH₄)₂-EDTA) oder einem Salz eines oder mehrerer der oben Genannten; oder
b) Diethylentriaminpentaessigsäure/Pentensäure (DTPA), Pentanatriumpentetat, Calciumtrinatriumpentetat, Nitrilotriessigsäure (NTA), Aminotris(methylenphosphonsäure) (ATMP), Ethylendiamintetramethylenphosphonsäure (EDTMP), Ethylendiamindisuccinsäure (EDDS), Iminodibernsteinsäure (IDS) oder einem Salz einer oder mehrerer der obigen Säuren; oder
c) einem Gemisch von zwei oder mehreren der obigen Säuren oder Salze davon, ausgewählt aus (a) oder (b);
wobei die amorphe Form frei von irgendwelchen zusätzlichen Verbindungen oder einer Trägermatrix ist.

2. Amorphe Form nach Anspruch 1, wobei der Chelatbildner Ethylendiamintetraessigsäure/Edetinsäure (EDTA), Natriumedetat (Na-EDTA), Dinatriumedetat (2Na-EDTA), Dikaliumedetat (2K-EDTA), Calciumdinatriumedetat (2NaCa-EDTA), Trinatriumedetat (3Na-EDTA), Diammoniumedetat ((NH₄)₂-EDTA), Diethylentriaminpentaessigsäure/Pentensäure (DTPA), Pentanatriumpentetat oder Calciumtrinatriumpentetat oder ein Gemisch von zwei oder mehreren der obigen Säuren oder Salze umfasst.

3. Verfahren zum Herstellen einer amorphen Form eines Chelatbildners nach einem der vorhergehenden Ansprüche, wobei das Verfahren die folgenden Schritte umfasst:
a) Lösen des Chelatbildners in einem geeigneten Lösungsmittel, um eine Lösung zu bilden;
b) optionale Reinigung der Lösung;
c) Isolieren einer amorphen Form des Chelatbildners, wobei die amorphe Form frei von irgendwelchen zusätzlichen Verbindungen oder einer Trägermatrix ist;
d) Optional Nachtrocknen oder Konditionieren der amorphen Form des Chelatbildners.

4. Verfahren nach Anspruch 3, wobei die Lösung unter Verwendung eines oder mehrerer Harze, Aktivkohle oder durch Filtration gereinigt wird.

5. Verfahren nach einem der Ansprüche 3 oder 4, wobei das Lösungsmittel Wasser, eine wässrige Lösung von Natriumhydroxid, eine wässrige Lösung von Ammoniak oder ein Gemisch aus zwei oder mehreren dieser Lösungsmittel ist.

6. Verfahren nach Anspruch 5, wobei ein organisches Lösungsmittel zugegeben werden kann, wobei das zusätzliche Lösungsmittel optional ein Alkohol oder Aceton ist.

7. Verfahren nach einem der Ansprüche 3 bis 6, wobei das Isolieren einer amorphen Form des Chelatbildners durch Lösungsmittelentfernung durchgeführt wird.

8. Verfahren gemäß einem der Ansprüche 3 bis 7, **dadurch gekennzeichnet, dass** eine Lösungsmittelentfernungstechnik wie Gefriertrocknung, Vakuumtrocknung oder Sprühtrocknung verwendet wird.

9. Verfahren nach einem der Ansprüche 3 bis 8, wobei ein Nachtrocknungs- oder Konditionierungsschritt durchgeführt wird.

10. Verfahren nach einem der Ansprüche 3 bis 9, wobei die Teilchengrößenverteilung des amorphen Chelatbildners gesteuert wird.

11. Verwendung eines amorphen Chelatbildners nach einem der Ansprüche 1 oder 2 in einem Verfahren oder einer Zusammensetzung im industriellen, landwirtschaftlichen oder Haushaltsbereich.

12. Verwendung nach Anspruch 11, wobei das Feld umfasst: Zellstoffbleichen, Lebensmittel- und Getränkekonservierung, industrielle Reinigung, Detergentien, Zement, Körperpflege und Kosmetika, Düngemittel, Wasserbehandlung oder Entfernung und Deaktivierung von Metallionen in Textilien.

13. Pharmazeutische oder biopharmazeutische Zusammensetzung, umfassend:
a) einen pharmazeutischen Wirkstoff, und
b) einen amorphen Chelatbildner nach einem der Ansprüche 1 oder 2.

## Revendications

1. Forme amorphe d'un agent chélatant choisi parmi :
a) acide éthylènediaminetétraacétique/acide édétique (EDTA), édétate de sodium (Na-EDTA), édétate disodique (2Na-EDTA), édétate dipotassique (2K-EDTA), édétate disodique de calcium (2NaCa-EDTA), édétate trisodique (3Na-EDTA) ou édétate de diammonium ((NH4)2-EDTA), ou un sel de l'un quelconque des un ou plusieurs des éléments ci-dessus ; ou
b) acide diéthylènetriaminepentaacétique/acide pentétique (DTPA), pentétate de pentasodium, pentétate de calcium trisodique, acide nitrilotriacétique (NTA), aminotris(acide méthylène phosphonique) (ATMP), acide éthylènediamine tétraméthylène phosphonique (EDTMP), acide éthylènediaminedisuccinique (EDDS), acide iminodisuccinique (IDS), ou un sel quelconque parmi les un ou plusieurs des acides ci-dessus ; ou
c) un mélange de deux ou plus quelconques par les acides ci-dessus ou de leurs sels choisis parmi (a) ou (b) ;
dans laquelle la forme amorphe est exempte de tout composé supplémentaire ou d'une matrice de support.

2. Forme amorphe selon la revendication 1, dans laquelle l'agent chélatant comprend acide éthylènediaminetétraacétique/acide édétique (EDTA), édétate de sodium (Na-EDTA), édétate disodique (2Na-EDTA), édétate dipotassique (2K-EDTA), édétate disodique de calcium (2NaCa-EDTA), édétate trisodique (3Na-EDTA) ou édétate de diammonium ((NH₄)₂-EDTA), acide diéthylènetriaminepentaacétique/acide pentétique (DTPA), pentétate de pentasodium, pentétate de calcium trisodique ou un mélange de deux ou plus quelconques parmi les acides et sels ci-dessus.

3. Procédé de préparation d'une forme amorphe d'un agent chélatant selon l'une quelconque des revendications précédentes, lequel procédé comprend les étapes suivantes :
a) la dissolution de l'agent chélatant dans un solvant approprié pour former une solution ;
b) facultativement la purification de la solution ;
c) l'isolation d'une forme amorphe de l'agent chélatant, dans lequel la forme amorphe est exempte de tout composé supplémentaire ou d'une matrice de support ;
d) facultativement, après séchage ou conditionnement de la forme amorphe de l'agent chélateur.

4. Procédé selon la revendication 3, dans lequel la solution est purifiée en utilisant une ou plusieurs résines, du charbon actif ou par filtration.

5. Procédé selon l'une quelconque des revendications 3 ou 4, dans lequel le solvant est : de l'eau, une solution aqueuse d'hydroxyde de sodium, une solution aqueuse d'ammoniaque, ou un mélange de deux ou plusieurs parmi ces solvants.

6. Procédé selon la revendication 5, dans lequel un solvant organique peut être ajouté, facultativement dans lequel le solvant supplémentaire est un alcool ou l'acétone.

7. Procédé selon l'une quelconque des revendications 3 à 6, dans lequel l'isolement d'une forme amorphe de l'agent chélatant est effectué par élimination du solvant.

8. Procédé selon l'une quelconque des revendications 3 à 7, dans lequel on utilise une technique d'élimination de solvant telle que la lyophilisation, le séchage sous vide ou le séchage par pulvérisation.

9. Procédé selon l'une quelconque des revendications 3 à 8, dans lequel une étape post séchage ou conditionnement est réalisée.

10. Procédé selon l'une quelconque des revendications 3 à 9, dans lequel la distribution granulométrique de l'agent chélatant amorphe est régulée.

11. Utilisation d'un agent chélatant amorphe selon l'une quelconque des revendications 1 ou 2 dans un procédé ou une composition dans le domaine industriel, agricole ou domestique.

12. Utilisation selon la revendication 11, dans laquelle le domaine comprend : le blanchiment de la pâte, la conservation agroalimentaire, le nettoyage industriel, les détergents, le ciment, les soins personnels et cosmétiques, les engrais, le traitement de l'eau, ou l'élimination et la désactivation des ions métalliques dans les textiles.

13. Composition pharmaceutique ou biopharmaceutique comprenant :
a) un ingrédient pharmaceutique actif, et
b) un agent chélatant amorphe selon l'une quelconque des revendications 1 ou 2.
